# EUROPEAN PATENT APPLICATION

(11) **EP 4 674 953 A1**
(43) Date of publication of application: **07.01.2026**
(21) Application number: 24819422.7
(22) Date of filing: 07.06.2024
(51) Int. Cl.: C12N 5/077, C12M 3/00, C12N 5/0775, C12Q 1/02

(54) **ARTIFICIAL TISSUE TUBE USING STEM CELL, AND METHOD FOR PRODUCING SAME**

(30) Priority: 08.06.2023 JP 2023094561
(71) Applicant: Tohoku University, Sendai-shi, Miyagi 980-8577 (JP)
(72) Inventor: ITAI, Shun, Sendai-shi, Miyagi 980-8577 (JP); TOYOHARA, Takafumi, Sendai-shi, Miyagi 980-8577 (JP)
(74) Representative: Dr. Schön, Neymeyr & Partner Patentanwälte mbB
(86) International application number: PCT/JP2024/020932
(87) International publication number: WO 2024/253195

(57) **Abstract**

The present inventions is to produce a tissue engineered tube including a smooth muscle layer and an endothelial cell layer derived from stem cells. It is possible to produce a tissue engineered tube that generates physiological contraction and relaxation reactions by producing a tissue engineered tube using smooth muscle cells derived from stem cells at least three times or more as those used in a method of the related art, and using endothelial cells derived from stem cells. Since an artificial blood vessel produced using iPS cells derived from a Werner syndrome patient indicates being an artificial blood vessel reflecting a pathological condition, tissue engineered tubes reflecting pathological conditions of various diseases can be produced using iPS cells. The tissue engineered tube can be used in pharmaceutical screening and transplantation.

## Description

### Technical Field

The present invention relates to a tissue engineered tube used for artificial blood vessels produced using cultured cells, particularly stem cells, and a method for producing the same.

### Background Art

An attempt has been made to construct an artificial three-dimensional tissue simulating the functions of a biological tissue. In a case where a three-dimensional tissue can be artificially constructed in vitro, the three-dimensional tissue can be expected to be used in many regions such as artificial organs using the cells of patients themselves and tests for new drug development instead of animal experiments. Various artificial tissues have been developed, one of which is the development of tissue engineered tube. The tissue engineered tube is a tubular structure artificially produced and including a cell tissue, and is a tissue artificially reproducing a tubular biological structure that contracts and relaxes, such as blood vessels, bronchi and intestines. In particular, since cardiovascular diseases affect a large number of patients, it is expected to develop more functional artificial blood vessels and develop models reflecting biological functions that can be used for screening pharmaceuticals using artificial blood vessels.

It is said that cardiovascular diseases (CVD) occupy about 1/3 of the cause of death throughout the world. Unfortunately, development of therapeutic agents and therapeutic methods for diseases secondarily induced by cardiovascular diseases, such as hypertension and diabetes, has been advanced, whereas no therapeutic method targeting malfunctions of a blood vessel itself has been developed yet. Since a blood vessel has a layered structure including a plurality of cells such as endothelial cells and smooth muscle cells, it is difficult to select a compound effective for malfunctions of a blood vessel, which is a tissue including a plurality of types of cells, in screening using cultured cells.

The usage of cardiovascular disease model mice has also been studied, which are not suitable for screening a large number of compounds, and are different in gene expression from humans and thus cannot completely reproduce the pathological conditions of humans observed in clinical practice. Therefore, it is desired to develop a blood vessel model that reproduces the structure of the blood vessel. Tissue engineered blood vessels (TEBV) derived from human cells have cells and structures constituting blood vessels, and thus are very useful as a model for elucidating the mechanism leading to CVD and screening therapeutic agents. Furthermore, a blood vessel model produced using stem cells can be used for producing a model for analyzing drug efficacy or for transplantation.

Several TEBVs using vascular smooth muscle cells (VSMCs) and endothelial cells (ECs) have already been established (PTL 1 and NPLs 1 and 2). PTL 1 discloses a multilayer structure obtained by laminating a plurality of layers having a tubular structure including an extracellular matrix, and discloses that blood vessels can be reproduced. In NPL 1, a blood vessel model is produced from iPS cells derived from a Hutchinson-Gilford progeria syndrome patient to analyze markers and pathological conditions of a cardiovascular disease. NPL 2 is a document by the inventors, and discloses that a blood vessel model is produced using human umbilical artery smooth muscle cells (HUASMCs) and human umbilical vein endothelial cells (HUVECs) as smooth muscle cells.

### Citation List

### Patent Literature

PTL 1: WO2018/207783

### Non Patent Literature

NPL 1: Atchison, L. et al., Stem Cell Reports, 2020, Vol. 14, pp. 325-337
NPL 2: Itai, S. & Onoe, H., 2022 IEEE 35th International Conference on Micro Electro Mechanical Systems Conference (MEMS), 2022, 10.1109/MEMS51670.2022.9699803
NPL 3: Tsujimoto, H. et. al., Cell Rep., 2020, 31, 10746
NPL 4: Novella, S. et al., Age 2013, Vol. 35(1), pp. 117-128.

### Summary of Invention

### Technical Problem

Unfortunately, in the invention described in PTL 1, cell tissues formed from adventitia cells and smooth muscle cells on a tubular support are seeded and fixed on a hard scaffold formed of collagen or the like, and thus cannot imitate deformation reactions of organs in a living body such as contraction and expansion. In the method disclosed in NPL 1, smooth muscle cells and endothelial cells are induced to differentiate from iPS cells. The smooth muscle cells are fixed in a collagen gel and the endothelial cells are applied to produce a blood vessel by tissue engineering. This method does not perform a treatment for orienting smooth muscle cells, and thus has no orientation in the circumferential direction of smooth muscle cells. In addition, contraction is observed, whereas the contraction is very weak. The degree of contraction is also weak in the method disclosed in NPL 2. It is well known that contraction and expansion play important roles in cardiovascular diseases, and artificial blood vessels capable of reproducing physiological contraction and expansion are required. It is also very useful for screening pharmaceuticals and developing therapeutic methods if possible to artificially produce not only blood vessels but also tissues that repeat contraction and relaxation by a smooth muscle layer, such as bronchi and intestines.

The production of artificial tissues using stem cells, particularly iPS cells, is useful for pharmaceutical screening and the elucidation of the pathogenic mechanism of diseases. The usage of iPS cells produced from patient cells enables only the transplantation to the patient but also the pharmaceutical screening suitable for specific diseases. Unfortunately, a tissue engineered tube using iPS cells has been developed, whereas a tissue engineered tube having a sufficient function, that is, a contraction force similar to that of a blood vessel of a living body, and capable of repeated contraction and expansion has not yet been developed.

An object of the invention is to provide a method for producing tissue engineered tube having a physiological function as a tissue or an organ using cultured human cells, particularly stem cells. Specifically, an object is to provide a tissue engineered tube that contracts and expands under physiological conditions, in particular, an artificial blood vessel. Another object is to provide a method for screening a compound that exhibits an effect on CVD.

### Solution to Problem

The invention relates to a tissue engineered tube, a method for producing the same, and a screening method using the same as described below.
(1) A tissue engineered tube including a smooth muscle layer containing smooth muscle cells wound in a spiral shape or an annular shape and having a density of 1.0 × 10⁸ cells/cm³ or more and 1.0 × 10¹⁰ cells/cm³ or less, in which the smooth muscle cells are derived from stem cells.
(2) The tissue engineered tube according to (1), further including:
   an extracellular matrix layer containing an extracellular matrix material present on an outer periphery of the smooth muscle layer.
(3) The tissue engineered tube according to (2), in which
   an inner wall of the smooth muscle layer contains endothelial cells derived from stem cells.
(4) The tissue engineered tube according to (1), in which
   the tissue engineered tube is for contracting and relaxing repeatedly.
(5) The tissue engineered tube according to (1), in which
   the tissue engineered tube is an artificial blood vessel.
(6) A method for producing a tissue engineered tube including a smooth muscle derived from stem cells, the method for producing tissue engineered tube including:
   producing a mold for producing a smooth muscle including a first groove for forming a smooth muscle fiber and a second groove intersecting the first groove;
   a core rod installation step of installing a core rod in the second groove;
   a step of installing an anchor on a side in the first groove opposite to a side where the second groove is provided;
   a step of pouring a pregel liquid including smooth muscle cells into a region excluding the periphery of the core rod of the first groove, and solidifying a gel;
   a step of pouring a pregel liquid not including smooth muscle cells in the periphery of the core rod of the first groove, and joining and solidifying the core rod and the gel including smooth muscle cells;
   a smooth muscle tissue acquisition step of culturing the smooth muscle cells to obtain a fibrous smooth muscle tissue; and
   a smooth muscle layer production step of winding the fibrous smooth muscle tissue around the core rod to produce a smooth muscle layer.
(7) The method for producing tissue engineered tube according to (6), in which
   the smooth muscle layer production step is a step of winding the fibrous smooth muscle tissue in a spiral shape around the core rod while the core rod is inclined.
(8) The method for producing tissue engineered tube according to (6), further including:
   an extracellular matrix layer forming step of, after the smooth muscle layer production step, immersing the smooth muscle layer in a liquid containing an extracellular matrix material to form an extracellular matrix layer.
(9) The method for producing tissue engineered tube according to (8), further including:
   an endothelial cell seeding step of seeding endothelial cells on an inner wall of the smooth muscle layer.
(10) A drug screening method using a tissue engineered tube having a smooth muscle derived from stem cells, the drug screening method including using the tissue engineered tube according to any one of (1) to (5) to perform:
   injecting a candidate compound into a lumen of the tissue engineered tube;
   measuring a biological reaction of the tissue engineered tube; and
   selecting the candidate compound.
(11) The drug screening method according to (10), in which
   the stem cells are cells derived from a patient.

### Brief Description of Drawings

[FIG. 1] FIG. 1 is a schematic view of a cross-sectional structure of an tissue engineered tube according to an embodiment of the invention.
[FIG. 2] FIG. 2 illustrates a perspective view (top), a top view (middle), and a side view (bottom) of a mold for producing smooth muscle fibers (first mold).
[FIG. 3A] FIG. 3A is a diagram schematically illustrating a step of producing smooth muscle fibers.
[FIG. 3B] FIG. 3B is a diagram schematically illustrating a step of winding the smooth muscle fibers.
[FIG. 4] FIG. 4 schematically illustrates a method for producing a tissue engineered tube containing endothelial cells and an extracellular matrix.
[FIG. 5] FIG. 5 is a graph illustrating the results of confirming the formation of the smooth muscle fibers by volume contraction.
[FIG. 6] FIG. 6 illustrates the results of confirming the formation of smooth muscle fibers by the orientation of actin filaments.
[FIG. 7] FIG. 7 illustrates the results of confirming a phase-contrast microscope image of the produced tissue engineered tube and confirming a section by hematoxylin-eosin staining, extracellular matrix staining, and anti-smooth muscle antibody immunostaining.
[FIG. 8A] FIG. 8A is a diagram illustrating the results of analyzing the contractile ability against a physiologically active substance by the presence or absence of endothelium of the produced artificial blood vessel.
[FIG. 8B] FIG. 8B is a diagram illustrating the results of analyzing the contractile ability against a physiologically active substance after the perfusion culture of the produced artificial blood vessel.
[FIG. 8C] FIG. 8C is a diagram illustrating concentration-dependent contraction of the artificial blood vessel due to U46619 and endothelin-1.
[FIG. 9A] FIG. 9A is a diagram illustrating the results of analyzing the contractile ability against a physiologically active substance by producing artificial blood vessels using iPS cells derived from healthy subjects and derived from Werner syndrome patients.
[FIG. 9B] FIG. 9B is a diagram illustrating the results of analyzing the relative contraction rate due to U46619 and angiotensin II using iPS cells derived from healthy subjects and derived from Werner syndrome patients.
[FIG. 9C] FIG. 9C is a diagram illustrating the results of analyzing the contraction against U46619 after the perfusion culture of artificial blood vessels produced using iPS cells derived from healthy subjects and derived from Werner syndrome patients.
[FIG. 10] FIG. 10 is a diagram illustrating the results of measuring the compliance by producing an artificial blood vessel using iPS cells derived from healthy subjects and derived from Werner syndrome patients.
[FIG. 11] FIG. 11 is a diagram illustrating the results of measuring the expansion and contraction of an artificial blood vessel due to pulse pressure. The upper part is a diagram illustrating an example of measurement results, and the lower part is a diagram illustrating the relative expansion ratio due to pulse pressure of artificial blood vessels using iPS cells derived from healthy subjects and derived from Werner syndrome patients.
[FIG. 12] FIG. 12 illustrates a volcano plot illustrating the results of metabolome analysis of artificial blood vessels produced from iPS cells derived from healthy subjects and derived from Werner syndrome patients (left), and illustrates metabolites having particularly large differences (right).
[FIG. 13] FIG. 13 is a diagram illustrating the expression of p16 in endothelial cells and smooth muscle cells derived from healthy subjects and derived from Werner syndrome patients.
[FIG. 14] FIG. 14 is a diagram schematically illustrating an example of a screening method using an artificial blood vessel.
[FIG. 15] FIG. 15 is a diagram schematically illustrating an example of a screening method using a plurality of artificial blood vessels.

### Description of Embodiments

In the present specification, a tissue engineered tube refers to a tubular structure artificially produced and including a cell tissue, and artificially reproduces a tubular biological structure that contracts and relaxes, such as blood vessels, bronchi and intestines. This also includes a configuration in which a connector or the like is joined to enable perfusion, or a structure including endothelial cells or an extracellular matrix and closer to a biological tissue. The terms " tissue engineered tube" and "artificial tissue model" can be used interchangeably, and "tissue engineered blood vessel (TEBV)" can be used interchangeably with the term "artificial blood vessel". Stem cells refer to cells having self-replication ability and differentiation ability, such as iPS cells (induced pluripotent stem cells), ES cells (embryonic stem cells), somatic stem cells, and tissue stem cells. iPS cells are preferably used for pharmaceutical screening, the elucidation of pathological conditions, and the like, since cells derived from patients can be used. In addition, since iPS cell stocks having various HLA have been constructed for iPS cells, a tissue engineered tube produced using iPS cells of HLA homodonors can also be used for transplantation.

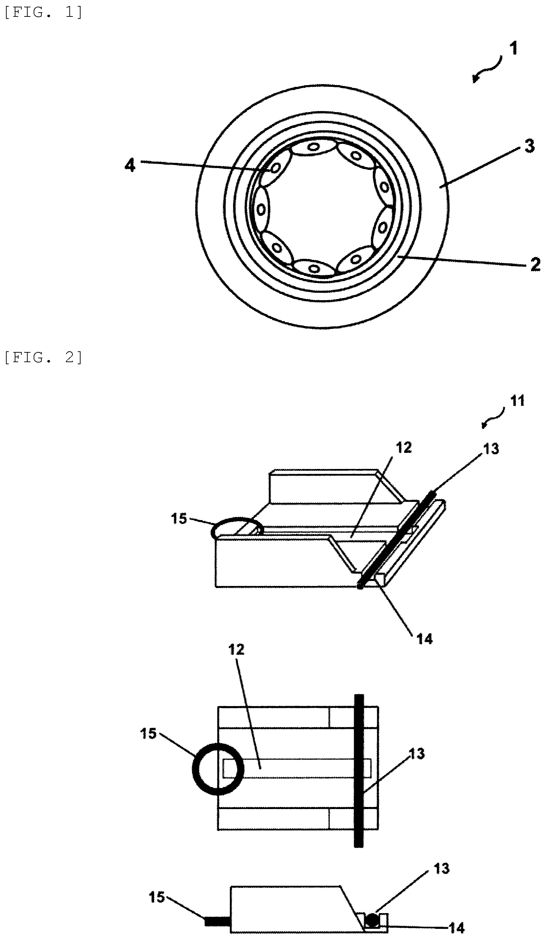

FIG. 1 schematically illustrates a cross-sectional structure of a tissue engineered tube. The tissue engineered tube 1 has a smooth muscle layer 2 including a smooth muscle wound in a spiral shape or annular shape. The inner diameter of the tubular biological structure is adjusted by contraction or relaxation of the smooth muscle. By arranging the smooth muscle cells in a spiral shape or an annular shape without a gap in the circumferential direction of the tissue engineered tube, the tissue engineered tube can imitate the contraction and relaxation of a biological tissue to faithfully reproduce the deformation reaction in a living body. The smooth muscle layer is preferably covered with an extracellular matrix layer 3 on the outer side, and the lumen surface thereof is preferably covered with endothelial cells 4 on the inner side. The smooth muscle layer 2 is formed from smooth muscle cells derived from stem cells. By using not only stem cells derived from healthy subjects but also stem cells derived from patients, it is possible to analyze the mechanism of the onset of a disease and to screen pharmaceuticals.

The smooth muscle layer 2 contains not only smooth muscle cells but also an extracellular matrix material. Examples of preferred extracellular matrix materials include, but are not limited to, collagen, laminin, gelatin, cadherin, hyaluronic acid, fibronectin, fibrin, elastin, chitin, chitosan, vitronectin, and proteoglycan. Among these extracellular matrix materials, collagen can be particularly preferably used. The extracellular matrix material may be used alone or in combination of two or more kinds thereof.

Further, it is preferable to have an extracellular matrix layer 3 on the outer periphery of the smooth muscle layer. The extracellular matrix layer 3 is a layer laminated on the side closer to the outer peripheral surface of the smooth muscle layer, and may include, for example, an extracellular matrix material and adventitia cells such as fibroblast cells. Examples of the extracellular matrix material include materials similar to the extracellular matrix material contained in the smooth muscle layer described above. The same material as or a different material from the extracellular matrix contained in the smooth muscle layer 2 may be used. The extracellular matrix layer 3 fills the space of the outer peripheral portion of the smooth muscle layer 2, thereby playing a role of a physical support and a role of a scaffold for adhering cells.

Furthermore, the inner wall of the smooth muscle layer 2 preferably contains the endothelial cells 4. The endothelial cells 4 are present on the side closer to the inner peripheral surface of the smooth muscle layer 2. In the tissue engineered tube, a plurality of endothelial cells are closely bonded to form a single layer. The endothelial cell layer can be formed by forming a tubular smooth muscle layer and then seeding endothelial cells on the inner wall thereof.

As will be described in detail below, the method for producing a tissue engineered tube of the invention is outlined as follows: the tissue engineered tube is produced by inducing smooth muscle cells from stem cells, forming smooth muscle fibers (smooth muscle tissue) from the smooth muscle cells by culture, and winding the smooth muscle tissue around a core rod. As illustrated in NPL 2, the present inventors have produced a tissue engineered tube by the same method. Unfortunately, the contraction force was weak, which causes a problem in producing a tissue engineered tube that contracts and expands repeatedly under physiological conditions. In the related art, 0.8 or 1.0 × 10⁶ cells/mL of cells have been used, which is equivalent to the number of cells used in the related studies. However, since the contraction force is insufficient, various conditions have been examined, and it has been clarified that it is important to adjust the number of cells such that the density is at least three times or more the number of cells used in the related art in order to produce a tissue engineered tube having a physiological contractile function, particularly an artificial blood vessel.

Further, to form an endothelial cell layer on the inner wall, endothelial cells are induced from stem cells, and the endothelial cells are seeded on the inner wall to form the endothelial cell layer. Although referred to as a tissue engineered tube regardless of the presence or absence of an endothelial cell layer, a tissue engineered tube having an endothelial cell layer is a model closer to a living body as shown by the data in the following.

### [Example 1]

### [Method for Producing Tissue Engineered Tube]

### (1) Method for Producing Smooth Muscle Fiber

A mold (first mold) for forming the smooth muscle tissue was produced by a 3D printer. FIG. 2 illustrates a perspective view, a top view, and a side view of the first mold. The mold was produced from polydimethylsiloxane (PDMS) here, but can also use materials usually used in this field such as polylactic acid (PLA). The first mold 11 has, in a flat plate-like substrate having a constant thickness, a first groove 12 for culturing smooth muscle cells to produce smooth muscle fibers, and a second groove 14 intersecting the first groove and being for installing a core rod 13 for winding the smooth muscle fibers positioned at an end of the first groove. In addition, the first groove is provided with, at the end opposite to the second groove, an anchor 15 used for winding the produced smooth muscle fibers in a fibrous shape. A circular anchor is exemplified here, whereas the anchor may have any shape, and a groove for installing the anchor may be provided in the mold.

The material constituting the core rod and the anchor is not particularly limited, and it is preferable to employ materials made of known metals employed in medical equipment, such as tungsten, stainless steel, titanium, aluminum, and niobium.

### (2) Preparation of Cell Suspension

Hereinafter, an example of producing a tissue engineered tube using iPS cells will be described, but ES cells, tissue stem cells, and the like can also be used for producing a tissue engineered tube by using a known culture method and differentiation induction method.

The tissue engineered tubes used in the following examples used iPS cells derived from healthy subjects and Werner syndrome patients. Undifferentiated iPS cells were cultured using a Stemflex culture solution, and the induction into smooth muscle cells and endothelial cells was performed based on the protocol of NPL 3. Briefly, for smooth muscle cells, first, the undifferentiated iPS cells were induced to a mesoderm using N2B27 medium (a mixed solution of Neurobasal medium, DMEM/F12 Glutamax medium, N2 supplement, B27 supplement, and 2-Mercaptethanol) and CHIR99021 and hBMP4, then induced to a smooth muscle using N2B27 medium, PDGF-BB, and Activin-A, and then maintained using type-I collagen coating, Smooth muscle growth medium (SmGM-2), M-231 medium, and Smooth muscle differentiation supplement (SMDS).

The endothelial cells were first induced to a mesoderm using DMEM/F12 Glutamax medium, B27 supplement, CHIR99021, hBMP4, Retinoic Acid (RA), and FGF-2, then induced to a blood vessel endothelium using Stempro medium, Laminin 411 coating, Y27632, DAPT, IWR-1, VEGF, hBMP4, and FGF-2, and then maintained using Fibronectin coating and Endodermal Growth Medium (EGM-2). For co-culture of vascular smooth muscle cells derived from iPS and blood vessel endothelial cells, a mixture of M231 medium (including SMDS) and EGM-2 at a volume ratio of 3:1 was used. Prior to cell seeding, the cells were cultured in a dish at 37°C under a humidity atmosphere of 5% CO₂, and the culture medium was replaced every two days. The differentiated cells derived from iPS cells were dispersed with a trypsin EDTA solution, the smooth muscle cells were suspended in a collagen pregel solution (a mixed solution of a Type-I acidic collagen solution, a reconstruction buffer solution (prepared from NaOH, NaHCO₃, and HEPES), and a 10X HANKS equilibrium salt solution), and the endothelial cells were suspended in EGM-2.

### (3) Formation of Smooth Muscle Fibers

A method for producing a tissue engineered tube is divided into a step of forming smooth muscle fibers, a winding step for obtaining a collagen tube in which a smooth muscle layer is embedded, and a step of covering the endothelial cells and the extracellular matrix. Details of the step of forming smooth muscle fibers are as follows.

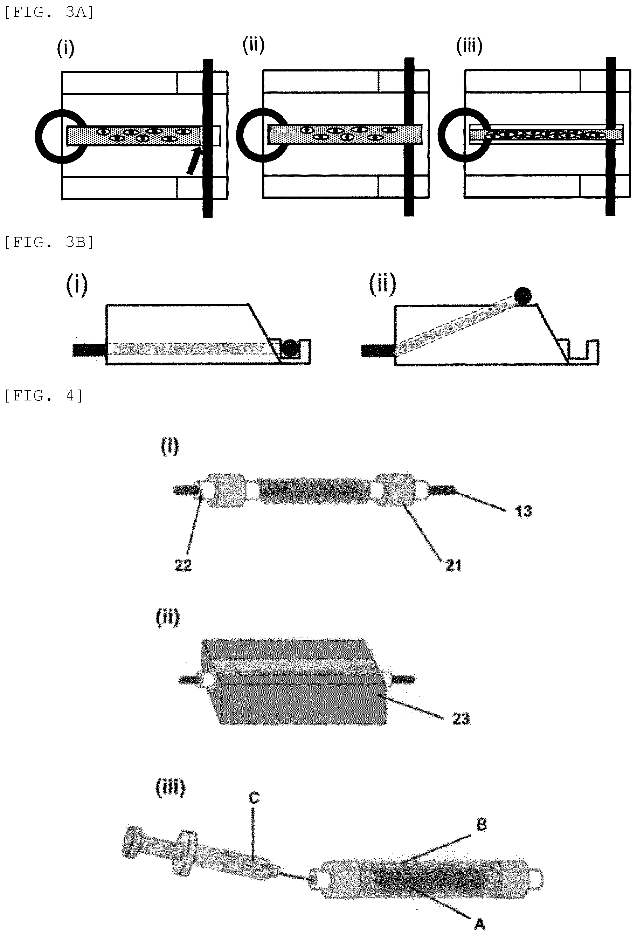

[1] A linear tungsten wire (core rod) and a circular tungsten wire (anchor) were placed in a first mold for fiber formation.
[2] A collagen pregel solution (1.0 mg/mL) in which iPS cells (1.0 × 10⁷ cells/mL) were suspended was poured into the first mold (FIG. 3A, (i)). At this time, the suspension was dropped around the core rod (in the region indicated by an arrow in the drawing) so as not to come into contact with the iPS cell suspension. This was because if smooth muscle cells were present around the core rod, the smooth muscle cells had a strong contraction force when the core rod was removed in the subsequent steps, making it difficult to remove the core rod. After being dropped, the cell suspension was incubated and solidified at 37°C.
[3] Only the collagen pregel solution was added around the core rod to join the core rod and the collagen gel (FIG. 3A, (ii)). The collagen was incubated and solidified at 37°C. Accordingly, since the core rod was bonded to the collagen gel containing no cells, the core rod was prevented from being applied a large force when the smooth muscle fiber was wound in the subsequent steps.

As shown in the following examples, by culturing in a mold, smooth muscle cells are oriented in the axial direction, and by culturing for about 24 hours, high-density fibers are formed and exert a strong contraction force. Here, smooth muscle cells formed at the number of cells at a density of 1.0 × 10⁷ cells/mL are mainly used, but it is presumed that a strong contraction force can be obtained when smooth muscle cells are formed at a density of 5.0 × 10⁶ cells/mL or more, preferably 7.0 × 10⁶ cells/mL or more, and more preferably 1.0 × 10⁷ cells/mL or more. In addition, when the number of cells exceeds 5.0 × 10⁸ cells/mL, smooth muscle cells are aggregated to hinder the formation of oriented tissue, and thus it is preferable to perform culture at a density of 5.0 × 10⁸ cells/mL or less, preferably 1.0 × 10⁸ cells/mL or less.

The present inventors have produced TEBV using cultured cells such as human umbilical artery smooth muscle cells (HUASMCs), and have produced smooth muscle fibers using iPS cells of 10 times or more as compared with the case of using these cultured cells. In the case of producing a tissue engineered tube at a cell density in the related art, even if the cell suspension is dropped into the first groove including the periphery of the core rod to produce smooth muscle fibers in a manner entangled with the core rod, no problem occurred in the subsequent steps. In the method of the related art, the number of cells used was small, so that the pressure due to contraction was not so large and the core rod could be removed. On the other hand, the number of cells used this time increased the cell density, so that the core rod could not be removed by the same method for producing. In order to remove the core rod in the subsequent steps, it is important to produce a collagen gel such that the core rod adhesive portion does not contain cells.

[4] The obtained tissue was cultured in a culture medium (M231 medium (+ SMDS)) for 24 hours to induce alignment in the axial direction by the cells per se, thereby forming dense fibers (FIG. 3 (A), iii). Although data will be described later, it was possible to confirm at this stage whether the smooth muscle fibers was produced appropriately by the volume of the fibers or the orientation of the actin filaments.

### (4) Production of Tissue Engineered Tube Embedded with Smooth Muscle Layer

The high-density smooth muscle fibers obtained above were wound around the core rod and embedded with collagen gel to produce a flexibly deformable collagen tube device with smooth muscle cells being arranged in the circumferential direction thereof (FIG. 3B is a side view of the mold).

[5] After the culture was complete (FIG. 3B, i), the tungsten wire (core rod) was rotated in the culture solution to wind the smooth muscle fibers around the wire. When the smooth muscle fibers were wound around the core rod, by winding the smooth muscle fibers at a predetermined inclination angle, the smooth muscle fibers could be avoided from overlapping and could be spontaneously wound around the core rod in a spiral shape. Since the side surface portion of the first mold had an inclined shape, by rolling the core rod along the inclination, the smooth muscle fibers could be wound in a spiral shape without overlapping (FIG. 3B, (ii)). Alternatively, the smooth muscle fiber may be wound using a base on which the core rod can be placed at an appropriate inclination.

[6] In seeding of endothelial cells, perfusion, addition of physiologically active substances, or the like, a connector or the like was joined to produce a tissue model. A connector including a silicone tube 21 and a glass capillary 22 was inserted into the tungsten wire (core rod 13) from both sides (FIG. 4 (i)).

[7] A collagen pregel solution (4 mg/mL) was poured around the smooth muscle layer and the connector using a second mold 23 to coat them with collagen. Thereafter, the collagen was solidified by incubation at 37°C (FIG. 4 (ii)).

[8] The second mold was removed to obtain a tissue engineered tube in which smooth muscle fibers A were arranged in the circumferential direction and a collagen gel B covers. Further, a tissue engineered tube including endothelial cells could be obtained by seeding endothelial cells C in the tube with a microsyringe (FIG. 4 (iii)).

### [Example 2]

### [Characteristics of Obtained Smooth Muscle Fibers]

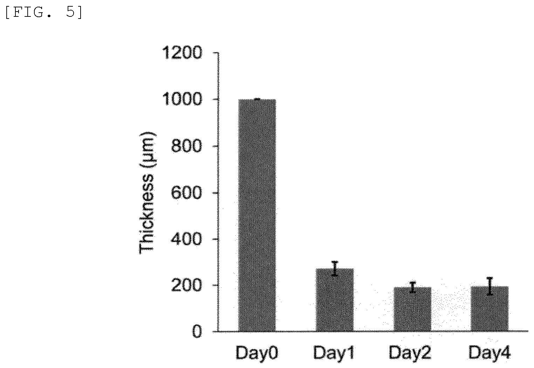

When cultured in the grooves of the mold, the vascular smooth muscle cells suspended in the collagen hydrogel were aligned in the axial direction to form high-density fibers. The degree of fiber formation was measured by measuring the width of fibers (the width of muscle fibers in a direction orthogonal to the long axis direction of fibers: thickness) with a phase-contrast microscope (FIG. 5). Human iPS cell 201B7 cell lines (obtained from RIKEN BRC) were induced into smooth muscle cells as cells derived from healthy subjects, and started being cultured in the grooves of the mold according to the above-described method for culturing smooth muscle fibers. After being cultured for 24 hours, the width of the muscle fibers decreased significantly due to contraction. This corresponded to a decrease in volume ratio of 95% as compared with that immediately after the start of culture (day 0). In this example, since culture was started at a density of 1.0 × 10⁷ cells/mL, muscle fibers having a density of 2.0 × 10⁸/cm³ were formed. This also well matched the results obtained from a pathological slice image described later.

Since the cells did not proliferate after being induced to differentiate into smooth muscle cells, the cell density was maintained at the same level after the production of the tissue engineered tube. As described above, a cell density of about 1 × 10⁸ cells/cm³ was obtained in a case where culture was started at a cell density of 5.0 × 10⁶ cells/mL, about 1.4 × 10⁸ cells/cm³ in a case where culture was started at a cell density of 7.0 × 10⁶ cells/mL, and about 2.0 × 10⁸ cells/cm³ in a case where culture was started at a cell density of 1.0 × 10⁷ cells/mL. Furthermore, a cell density about 2 × 10⁹ cells/cm³ was obtained in a case where the culture was started at a cell density of 1.0 × 10⁸ cells/mL, and a cell density of about 1 × 10¹⁰ cells/cm³ in a case where the culture was started at a cell density of 5.0 × 10⁸ cells/mL. It was important to start the culture with the cells being adjusted to have a density of 1 × 10⁸ cells/cm³ or more and 1 × 10¹⁰ cells/cm³ or less when forming smooth muscle fibers.

### [Example 3]

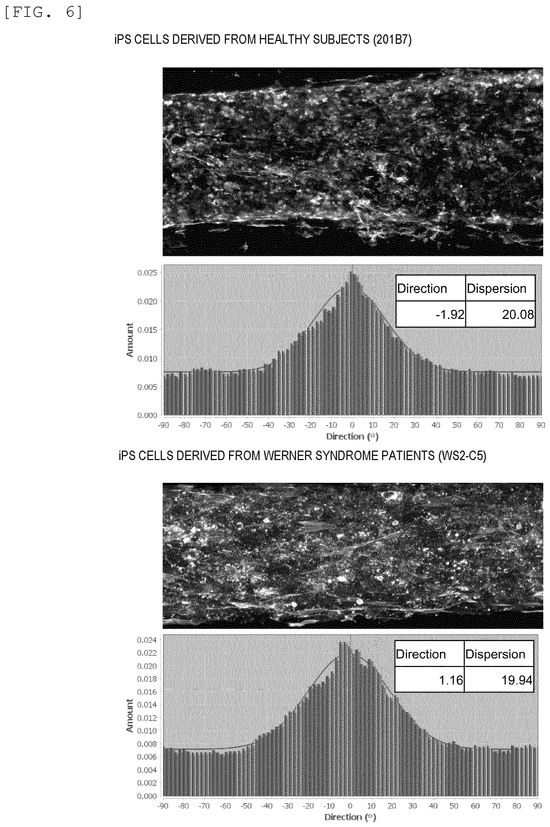

Muscle fibers were produced using 201B7 cells and iPS cell lines iPS-WS2-C5 derived from Werner syndrome patients (obtained from RIKEN BRC), and the orientation of actin filaments thereof was observed. The orientation of the actin filaments of the produced smooth muscle fibers was observed by staining the actin fibers with SPY555-actin (Cytoskeletonon Inc.) and using a laser confocal microscope (FIG. 6). The quantitative analysis results of the direction and dispersion of each fiber are illustrated in the lower part of each micrograph, and the numerical values in the graph indicate the average values thereof. It was confirmed that in the case of using either cell line, the direction of the fiber was a value close to 0, and the dispersion thereof was small, so that the actin filaments were oriented in the long axis direction of the groove of the mold. This shows that there is no difference in the shapes of the smooth muscle fibers between the iPS cells derived from the healthy subjects and the iPS cells derived from Werner syndrome patients.

Werner syndrome is an autosomal recessive inherited disease, and is one of progeria in which an aging sign is observed early. In addition to typical changes such as cataracts and hair changes such as white hair and hair loss, it is known to cause arteriosclerosis in the early stage and to cause angina pectoris, myocardial infarction, and the like. Therefore, artificial blood vessels produced from iPS cells derived from Werner syndrome patients are considered to be arteriosclerosis models.

### [Example 4]

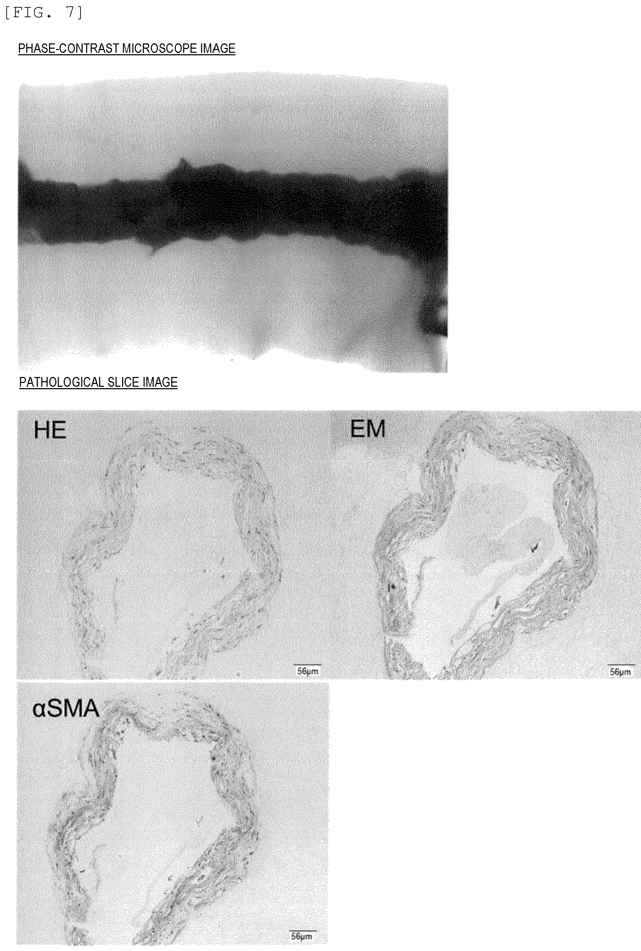

FIG. 7 illustrates a phase-contrast microscope image and a pathological slice image of the produced tissue engineered tube. It was observed from the phase-contrast microscope image that the muscle fibers were wound in the circumferential direction, and it was confirmed that the muscle fibers were oriented in the circumferential direction of the smooth muscle layer by staining with hematoxylin-eosin (HE), staining of extracellular matrices such as collagen fibers and elastic fibers (staining with EM: resorcin-fuchsin solution, hematoxylin solution, phosphotungstic acid / phosphomolybdic acid mixed solution, or light green solution), and staining with an anti-α-smooth muscle cell actinin (αSMA). All of the illustrated microscopic images are images of tissue engineered tubes obtained from 201B7 cells derived from healthy subjects, but similar images were observed for tissue engineered tubes obtained from WS2-C5 cells derived from Werner syndrome patients. In addition, it has been confirmed that a tissue engineered tube produced from any cell line repeats contraction and relaxation under a phase-contrast microscope.

### [Example 5]

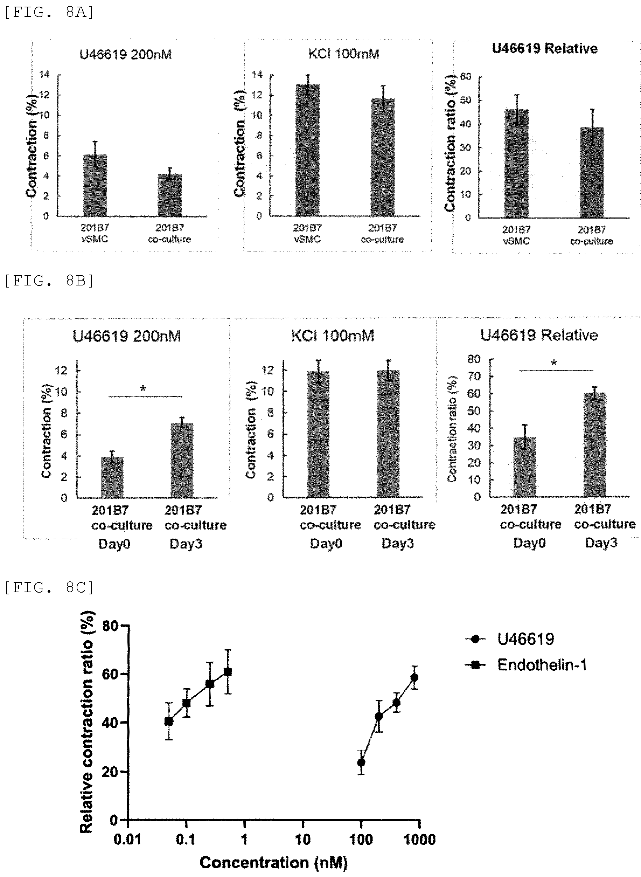

Next, it was analyzed whether the produced tissue engineered tube reproduces physiological reactions such as contraction and expansion with respect to a physiologically active substance. First, regarding artificial blood vessels produced from 201B7 cells, an artificial blood vessel of only smooth muscle cells (201B7 vSMC) and an artificial blood vessel obtained by seeding and co-culturing blood vessel endothelial cells induced to differentiate from 201B7 in an artificial blood vessel lumen (201B7 co-culture) were produced, and the contraction reaction due to U46619 and KCl was analyzed (FIG. 8A). U46619 is a stable synthetic analog of prostaglandin PGH2 and has an action of inducing blood vessel contraction as a thromboxane A2 receptor agonist. KCl is known to induce blood vessel contraction by membrane potential change. Since an individual difference may occur under a normal contraction amount, U46619 relative is a normalized value obtained by dividing the contraction amount when U46619 is applied by the contraction amount when KCl is applied, which indicates the maximum contraction force. Regardless of whether U46619 or KCl is applied, high contraction was observed in the artificial blood vessel produced from smooth muscle cells alone (201B7 vSMC). The reason is considered as that blood vessel endothelial cells do not produce nitrogen monoxide (NO). NO is a vasodilator and is known to be released from blood vessel endothelial cells. Therefore, it is recognized that the produced artificial blood vessel reproduces the physiological mechanism. Further, it has been confirmed that the same contraction reaction occurs not only in U46619 but also in endothelin-1 and angiotensin II, which are physiological vasoconstrictors. In the following experiments, artificial blood vessels co-cultured with endothelial cells were used unless otherwise specified.

In order to maintain a more physiological environment, perfusion culture was performed to examine whether the contraction rate was improved (FIG. 8B). Immediately after the start of perfusion culture, U46649 or KCl was applied to an artificial blood vessel produced from 201B7 cells (201B7 co-culture) on the third day of perfusion to analyze the contraction rate. In either case, an improvement in contraction rate due to perfusion culture was observed. It is known that blood vessel tissues change in phenotype, orientation, and the like and mature due to shear stress and blood pressure caused by perfusion culture. The improvement of the contraction rate by the perfusion culture indicates that the artificial blood vessel was matured by the perfusion culture.

It was analyzed whether the contraction of the artificial blood vessel with respect to U46619 and endothelin-1 depended on the concentration. Artificial blood vessels were produced using iPS cells derived from healthy subjects, and the contraction rates were measured while changing the concentrations of U46619 (0.05 nM to 0.5 nM) and endothelin-1 (100 nM to 1000 nM) (n = 5). The artificial blood vessels were contracted in a concentration-dependent manner in a physiological concentration range for either compound. Therefore, it is recognized that the produced artificial blood vessels has have physiological properties.

### [Example 6]

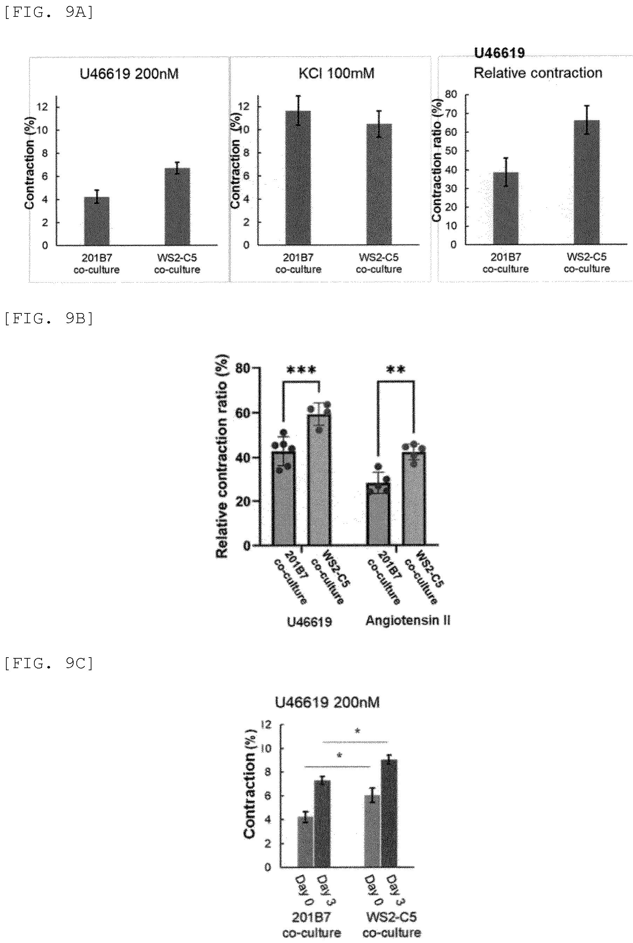

Next, reactions to U46619 and KCl were analyzed using artificial blood vessels produced from Werner syndrome patients iPS (FIG. 9A). iPS cells derived from healthy subjects and Werner syndrome patients were induced to differentiate into smooth muscle cells and blood vessel endothelial cells to produce artificial blood vessels as described above, and contraction with respect to U46619 and KCl was analyzed. In addition, similar to in Example 5, the relative contraction rate was calculated with the contraction amount when KCl was applied, which exhibits the maximum contraction force, as 100% (the right part in FIG. 9A). The artificial blood vessels produced from iPS cells derived from Werner syndrome patients (WS2-C5 co-culture) tended to contract more strongly against stimulation (n = 4) than artificial blood vessels produced from iPS cells derived from healthy subjects (201B7 co-culture). This result shows the same tendency as the result of the related studies using an accelerated aging mouse (SAMP8) (NPL 4).

In addition to U46619, the relative contraction rate was also determined for angiotensin II (FIG. 9B). The artificial blood vessel was contracted by 200 nM U46619 or 100 nM angiotensin II, and the relative contraction rate was calculated with the contraction amount when 100 mM KCl was applied as 100%. The artificial blood vessels produced using iPS cells derived from Werner syndrome patients (Werner derived iPSC blood vessels) exhibited stronger contraction force than artificial blood vessels produced using iPS cells derived from healthy subjects (WT derived iPSC blood vessels). Hereinafter, the artificial blood vessels produced using iPS cells derived from healthy subjects may be referred to as WT derived iPSC blood vessels, and the artificial blood vessels produced using iPS cells derived from Werner syndrome patients may be referred to as Werner derived iPSC blood vessels.

Artificial blood vessels were produced from iPS cells derived from healthy subjects and Werner syndrome patients and subjected to perfusion culture, and the contraction by U46619 was analyzed (FIG. 9C). Compared with the WT derived iPSC blood vessels, an increase in contraction force was observed in the Werner derived iPSC blood vessels. This is considered to be caused by a decrease in NO production ability of blood vessel endothelial cells derived from iPS cells of Werner syndrome patients. Therefore, it was suggested that an artificial blood vessel model simulating the pathological condition of Werner syndrome was produced.

### [Example 7]

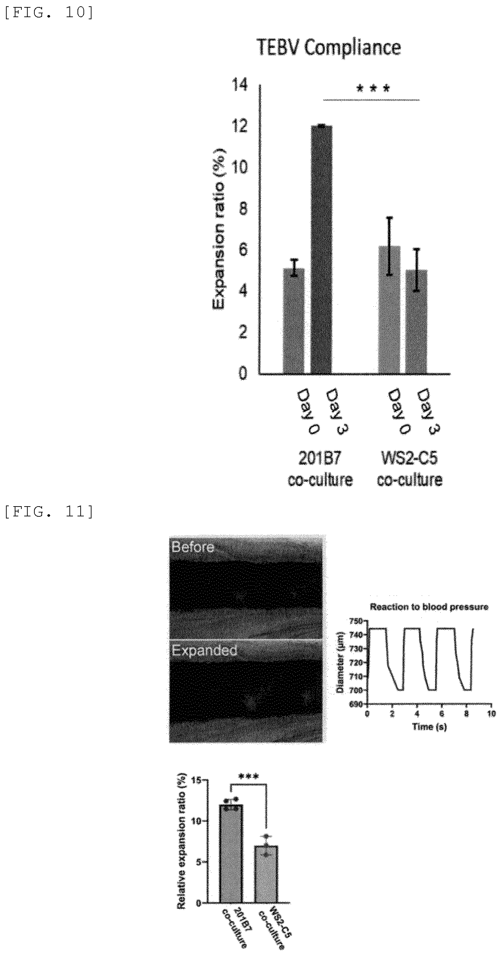

Perfusion culture of the artificial blood vessels was performed, and the compliance was measured (FIG. 10). Upon the perfusion, a pressure of 30 mmHg to 50 mmHg was applied to the produced artificial blood vessel having an inner diameter of about 0.5 mm. This pressure is equivalent to that of a living blood vessel having a similar diameter. A higher internal pressure can be applied when an artificial blood vessel having a larger diameter is used. The blood vessel compliance is an index of elasticity of a blood vessel, and the value thereof decreases with arteriosclerosis. In an artificial blood vessel having a smooth muscle layer and an endothelial cell layer produced from iPS cells derived from Werner syndrome patients, the compliance was significantly smaller than that of an artificial blood vessel produced from iPS cells derived from healthy subjects. This result also suggests that an artificial blood vessel model simulating the pathological condition of Werner syndrome was produced.

### [Example 8]

Similar to Example 7, the pressure corresponding to the pulse pressure was applied to the artificial blood vessels, the expansion and contraction of the blood vessels at that time were recorded (the upper left part of FIG. 11), and the change in the diameter of the artificial blood vessels due to the pressure change was recorded (the upper right part of FIG. 11). The expansion ratio (the ratio of the diameter increased when the pressure was applied to the diameter of the artificial blood vessel before the pressure was applied) in the WT derived iPSC blood vessel and the Werner derived iPSC blood vessel was determined (the lower part of FIG. 11). The expansion ratio of the blood vessel of the Werner derived iPSC blood vessel was significantly lower than that of the WT derived iPSC blood vessel. A result reflecting the pathological condition of Werner syndrome was obtained.

### [Example 9]

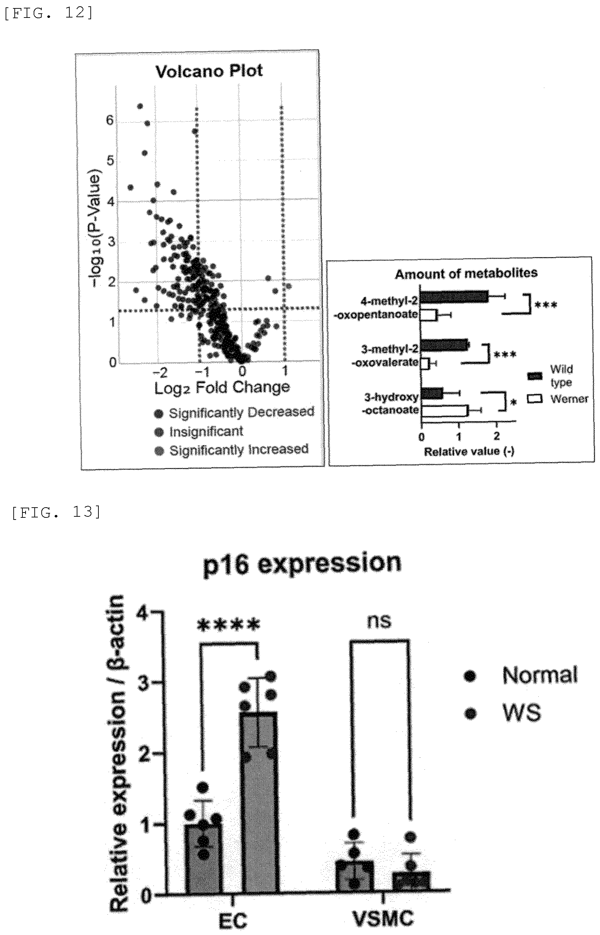

Metabolome analysis of WT derived iPSC blood vessels and Werner derived iPSC blood vessels was performed, and a large difference was observed between the metabolites of the two, which is illustrated by volcano plots (the left part in FIG. 12). The amounts of metabolites of Werner derived iPSC blood vessels relative to WT derived iPSC blood vessels are plotted. It was revealed that many metabolites were reduced in the Werner derived iPSC blood vessels. Those whose change is particularly large are illustrated on the right. The changes in the metabolites were consistent with the results of the related studies reported for Werner syndrome.

### [Example 10]

Next, iPS cells derived from healthy subjects and Werner syndrome patients were induced to differentiate into endothelial cells (EC) and smooth muscle cells (VSMC), and p16 expression was analyzed by RT-qPCR. As the results of normalization by β-actin expression and comparison, significant enhancement of p16 expression was observed in the endothelial cells differentiated by induction from the iPS cells derived from Werner syndrome patients. p16 has also been known as an aging marker, and it was confirmed that aging is promoted in the endothelium of Werner syndrome.

### [Use in Screening]

### [Example 11]

These results indicate that a tissue engineered tube produced from iPS cells derived from a patient is a good model reflecting the pathological condition. Although iPS cells of Werner syndrome patients are used here, it is needless to say that tissue engineered tubes simulating other diseases can be produced. Therefore, it is possible to screen drugs suitable for the pathological condition by producing a pathological condition model using iPS cells, adding drug candidate compounds to the culture solution, and measuring the physiological reaction. For example, since arteriosclerosis is caused by physiological changes in blood vessel endothelial cells and smooth muscle cells constituting a blood vessel, it is difficult to screen effective compounds by a screening method using one type of cells as in the related art. However, there is a possibility that an effective compound that acts on a tissue including a plurality of cells can be screened by using a pathological condition model reflecting a physiological reaction as shown in the present example.

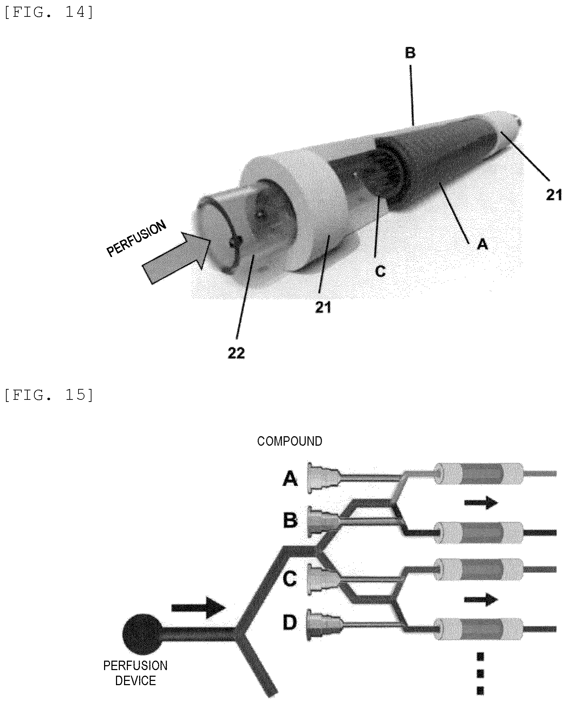

FIGS. 14 and 15 schematically illustrate a screening method for a compound using an artificial blood vessel. FIG. 14 schematically illustrates an artificial blood vessel. The artificial blood vessel is covered with collagen gel B on the outside of a smooth muscle layer including smooth muscle fibers A, and is covered with endothelial cells C on the inner wall. The artificial blood vessel has the silicone tube 21 and the glass capillary 22 attached to both ends and thus is easy to handle. Contraction and relaxation can be induced by perfusing a solution containing a physiologically active substance from a glass capillary. Furthermore, it is possible to analyze the effect of the candidate compound on the blood vessel function by confirming the effect on contraction and relaxation by adding the compound. As described in the above examples, it is possible to induce repeated contraction and relaxation.

FIG. 15 illustrates a method of simultaneously screening a plurality of compounds. The artificial blood vessel is connected by a perfusion device, and a culture solution, artificial blood, or the like is perfused. The candidate compound may be injected into each artificial blood vessel to analyze the properties of the blood vessel such as blood vessel contraction and compliance. By using iPS cells produced from cells derived from a patient, it is possible to screen pharmaceuticals suitable for each pathological condition. It is needless to say that screening can be performed by producing models of not only artificial blood vessels but also bronchi, intestines, and the like.

Many diseases are considered to be related to malfunctions of blood vessels, and are considered to be subject diseases of pharmaceutical screening using artificial blood vessels. Examples of such diseases include: arteriosclerosis including myocardial infarction, cerebral infarction, renal sclerosis, retinopathy; coronary contraction angina pectoris; diabetes (including pathological conditions in which blood vessels proliferate); vasculitis hypertension including various diseases such as ANCA-related vasculitis, Takayasu's arteritis, and IgG4 vasculitis; embolism, which is thrombus formation in blood vessels; blood vessel malformation disease (moyamoya disease); and genetic diseases (mitochondrial diseases). Artificial blood vessels can also be used for drug toxicity evaluation of drugs that cause blood vessel disorders, such as anticancer agents.

Furthermore, artificial blood vessels produced from iPS cells can also be used for transplantation. Artificial blood vessels produced using iPS cells of HLA homodonors can be transplanted as a substitute for blood vessels occluded and damaged due to diabetes or the like. As described in the above examples, the artificial blood vessel derived from iPS shows a physiological contraction reaction with respect to physiological vasoconstrictors. Arteriosclerosis may have progressed in the blood vessel of the subject, but an artificial blood vessel derived from iPS cells does not have such concern and thus can be suitably used as a blood vessel for transplantation.

### Reference Signs List

1: tissue engineered tube
2: smooth muscle layer
3: extracellular matrix layer
4: endothelial cell
11: first mold
12: first groove
13: core rod
14: second groove
15: anchor
21: silicone tube
22: glass capillary
23: second mold
A: smooth muscle fiber
B: collagen gel
C: endothelial cell

## Claims

1. A tissue engineered tube comprising a smooth muscle layer containing a smooth muscle tissue wound in a spiral shape or an annular shape and having a density of 1.0 × 10⁸ cells/cm³ or more and 1.0 × 10¹⁰ cells/cm³ or less, wherein
the smooth muscle tissue is derived from stem cells.

2. The tissue engineered tube according to claim 1, further comprising:
an extracellular matrix layer containing an extracellular matrix material present on an outer periphery of the smooth muscle layer.

3. The tissue engineered tube according to claim 2, wherein
an inner wall of the smooth muscle layer contains endothelial cells derived from stem cells.

4. The tissue engineered tube according to claim 1, wherein
the tissue engineered tube is for contracting and relaxing repeatedly.

5. The tissue engineered tube according to claim 1, wherein
the tissue engineered tube is an artificial blood vessel.

6. A method for producing a tissue engineered tube including a smooth muscle derived from stem cells, the method for producing tissue engineered tube comprising:
producing a mold for producing a smooth muscle including a first groove for forming a smooth muscle fiber and a second groove intersecting the first groove;
a core rod installation step of installing a core rod in the second groove;
a step of installing an anchor on a side in the first groove opposite to a side where the second groove is provided;
a step of pouring a pregel liquid including smooth muscle cells into a region excluding the periphery of the core rod of the first groove, and solidifying a gel;
a step of pouring a pregel liquid not including smooth muscle cells in the periphery of the core rod of the first groove, and joining and solidifying the core rod and the gel including smooth muscle cells;
a smooth muscle tissue acquisition step of culturing the smooth muscle cells to obtain a fibrous smooth muscle tissue; and
a smooth muscle layer production step of winding the fibrous smooth muscle tissue around the core rod to produce a smooth muscle layer.

7. The method for producing a tissue engineered tube according to claim 6, wherein
the smooth muscle layer production step is a step of winding the fibrous smooth muscle tissue in a spiral shape around the core rod while the core rod is inclined.

8. The method for producing tissue engineered tube according to claim 6, further comprising:
an extracellular matrix layer forming step of, after the smooth muscle layer production step, immersing the smooth muscle layer in a liquid containing an extracellular matrix material to form an extracellular matrix layer.

9. The method for producing tissue engineered tube according to claim 8, further comprising:
an endothelial cell seeding step of seeding endothelial cells on an inner wall of the smooth muscle layer.

10. A drug screening method using a tissue engineered tube having a smooth muscle derived from stem cells, the drug screening method comprising using the tissue engineered tube according to any one of claims 1 to 5 to perform:
injecting a candidate compound into a lumen of the tissue engineered tube;
measuring a biological reaction of the tissue engineered tube; and
selecting the candidate compound.

11. The drug screening method according to claim 10, wherein
the stem cells are cells derived from a patient.
